# EUROPEAN PATENT APPLICATION

(11) **EP 1 345 153 A2**
(43) Date of publication of application: **17.09.2003**
(21) Application number: 02025935.4
(22) Date of filing: 20.11.2002
(51) Int. Cl.: G06F 19/00

(54) **Health-related-information supplying method and apparatus**

(30) Priority: 12.03.2002 JP 2002066567
(71) Applicant: Colin Corporation, Komaki-shi, Aichi-ken (JP)
(72) Inventor: Oka, Tohru, Komaki-shi, Aichi-ken (JP); Narimatsu, Kiyoyuki, Komaki-shi, Aichi-ken (JP)
(74) Representative: Winter, Brandl, Fürniss, Hübner, Röss, Kaiser, Polte Partnerschaft

(57) **Abstract**

A method of supplying, from a server which is connected via a communication line to a plurality of member's terminal devices which are respectively operated by a plurality of members who are registered in advance in the server, at least one set of health-related information based on a set of medical information related to one of the members and stored in the server, to the one member, in response to a health-related-information request sent to the server from the one member, the method including a health-related-information-request identifying step of identifying the health-related-information request sent to the server from the one member, an information sending step of sending, when the health-related-information request sent from the one member has been identified, the health-related-information request sent from the one member, and the set of medical information related to the one member and stored in the server, to at least one dealer who is registered in advance in the server, in a manner that the one member is unidentified, so as to request the dealer to send the set of health-related information, and a health-related-information supplying step of receiving the set of health-related information sent from the dealer in response to the health-related-information request and the set of medical information sent to the dealer, and supplying the received set of health-related information to the one member.

## Description

### BACKGROUND OF THE INVENTION

### Field of the Invention

The present invention relates to a health-related-information supplying method and a health-related-information supplying apparatus each for supplying health-related information that is requested by each of a plurality of members and is produced by taking medical information of the each member into consideration.

### Related Art Statement

Health-related information, such as sporting goods or health equipments for improving health; supplements or other sorts of health food, or drugs; facilities where sporting goods or fields are offered; or rental videos which show dieting method or exercising methods, can be reached by reading books, seeing or hearing television or radio broadcasting, or referring to homepages through the internet,

However, the health-related information obtained as described above is for general purposes or wide uses only, and is not specific to an individual person. Thus, the conventional health-related information cannot be used as it is by the individual person. In addition, it is cumbersome and time-consuming to read books, hear broadcasting, or refer to homepages through internet.

### SUMMARY OF THE INVENTION

It is therefore an object of the present invention to provide a health-related-information supplying method and a health-related-information supplying apparatus each of which can easily and quickly supply health-related information suited for medical information of each of a plurality of members, in response to a request from the each member.

The above object has been achieved by the present invention. According to a first aspect of the present invention, there is provided a method of supplying, from a server which is connected via a communication line to a plurality of member's terminal devices which are respectively operated by a plurality of members who are registered in advance in the server, at least one set of health-related information based on a set of medical information related to one of the members and stored in the server, to the one member, in response to a health-related-information request sent to the server from the one member, the method comprising a health-related-information-request identifying step of identifying the health-related-information request sent to the server from the one member, an information sending step of sending, when the health-related-information request sent from the one member has been identified, the health-related-information request sent from the one member, and the set of medical information related to the one member and stored in the server, to at least one dealer who is registered in advance in the server, in a manner that the one member is unidentified, so as to request the dealer to send the set of health-related information, and a health-related-information supplying step of receiving the set of health-related information sent from the dealer in response to the health-related-information request and the set of medical information sent to the dealer, and supplying the received set of health-related information to the one member.

According to this invention, when one of the members sends, to the server, a request for one or more sets of health-related information, the health-related-information request sent from the one member, and the set of medical information related to the one member and stored in the server, are sent anonymously to one or more dealers, i.e., one or more terminal devices of the one or more dealers, via one or more electronic mails or an electronic bulletin. Then, when the set or sets of health-related information is or are received from the dealer or dealers in response to the health-related-information request and the set of medical information sent to the dealer or dealers, the received set of health-related information is supplied to the one member. Thus, the present method can easily and quickly supply the sets of health-related information suited for the medical information of each member, to the each member, in response to the request from the each member. In addition, since the medical information of each member is sent anonymously to the dealers, the secrecy of medical information of the each member is not damaged.

According to a preferred feature of the first aspect of the present invention, the health-related-information supplying method further comprises a medical-information supplying step of selecting, from a plurality of sets of medical information respectively related to the plurality of members and stored in the server, the set of medical information related to a set of physical information of the one member inputted through a corresponding one of the member's terminal devices, and sending the selected set of medical information, to the one member, and an information storing step of storing the set of physical information of the one member inputted through the one member's terminal device and adding the set of physical information to the selected set of medical information.

According to this feature, the set of physical information of the member inputted through the member's terminal device to obtain the set of medical information related to the member, and the set of medical information supplied to the member are stored. Thus, the respective sets of physical information, and the respective sets of medical information, of the members are easily stored, on one hand; and, when each member requests one or more sets of health-related information, the set of medical information including the set of physical information stored at the information storing step, is anonymously sent to the dealer, on the other hand. Thus, the more suitable set or sets of health-related information for the requesting member are prepared, and are supplied to the member.

According to another feature of the first aspect of the present invention, the health-related-information supplying method further comprises a diagnosing step of making a diagnosis about a physical condition of the one member based on the set of physical information of the one member sent to the server from the one member's terminal device, and the information sending step comprises sending, to the dealer, the selected set of medical information including the made diagnosis.

According to this feature, if each member inputs his or her physical information to obtain his or her medical information, then, at the diagnosing step, a diagnosis is automatically made based on the physical information of the each member, and the thus made diagnosis is sent with the set of medical information to the dealer. Thus, the more appropriate set of health-related information for the member is prepared, and is sent to the member.

According to a second aspect of the present invention, there is provided a medical-information supplying apparatus including a server which is connected via a communication line to a plurality of member's terminal devices which are respectively operated by a plurality of members who are registered in advance in the server, and supplying, from the server, a set of health-related information based on a set of medical information related to one of the members and stored in the server, to the one member, in response to a health-related-information request sent to the server from the one member, the apparatus comprising: a health-related-information-request identifying means for identifying the health-related-information request sent to the server from the one member; an information sending means for sending, when the health-related-information-request identifying means has identified the health-related-information request sent from the one member, the health-related-information request sent from the one member, and the set of medical information related to the one member and stored in the server, to at least one dealer who is registered in advance in the server, in a manner that the one member is unidentified, so as to request the dealer to send the set of health-related information, and a health-related-information supplying means for receiving the set of health-related information sent from the dealer in response to the health-related-information request and the set of medical information sent to the dealer by the information sending means, and supplying the received set of health-related information to the one member.

According to this invention, when one of the members sends, to the server, a request for one or more sets of health-related information, the health-related-information request sent from the one member, and the set of medical information related to the one member and stored in the server, are sent anonymously to one or more dealers, i.e., one or more terminal devices of the one or more dealers, via one or more electronic mails or an electronic bulletin. Then, when the set or sets of health-related information is or are received from the dealer or dealers in response to the health-related-information request and the set of medical information sent to the dealer or dealers, the received set of health-related information is supplied to the one member. Thus, the present method can easily and quickly supply the sets of health-related information suited for the medical information of each member, to the each member, in response to the request from the each member. In addition, since the medical information of each member is sent anonymously to the dealers, the secrecy of medical information of the each member is not damaged.

According to a preferred feature of the second aspect of the present invention, the health-related-information supplying apparatus further comprises a medical-information supplying means for selecting, from a plurality of sets of medical information respectively related to the plurality of members and stored in the server, the set of medical information related to a set of physical information of the one member inputted through a corresponding one of the member's terminal devices, and sending the selected set of medical information, to the one member; and an information storing means for storing the set of physical information of the one member inputted through the one member's terminal device and adding the set of physical information to the selected set of medical information.

According to this feature, the set of physical information of the member inputted to obtain the set of medical information related to the member, and the set of medical information supplied to the member are stored. Thus, the respective sets of physical information, and the respective sets of medical information, of the members are easily stored, on one hand; and, when each member requests one or more sets of health-related information, the set of medical information including the set of physical information stored by the information storing means, is sent to the dealer, on the other hand. Thus, the more suitable set or sets of health-related information for the requesting member are prepared, and are supplied to the member.

According to another feature of the second aspect of the present invention, the health-related-information supplying apparatus further comprises a diagnosing means of making a diagnosis about a physical condition of the one member based on the set of physical information of the one member sent to the server from the one member's terminal device, and the information sending means sends, to the selected instructor, the selected set of medical information including the made diagnosis.

According to this feature, if each member inputs his or her physical information to obtain his or her medical information, then, the diagnosing means automatically makes a diagnosis based on the physical information of the each member, and the information sending means sends the thus made diagnosis with the set of medical information, to the dealer. Thus, the more appropriate set of health-related information for the member is prepared, and is sent to the member.

### BRIEF DESCRIPTION OF THE DRAWINGS

The above and optional objects, features, and advantages of the present invention will be better understood by reading the following detailed description of the preferred embodiments of the invention when considered in conjunction with the accompanying drawings, in which:
Fig. 1 is a view for explaining the construction of a health-related-information supplying apparatus utilizing a communication line, to which the present invention is applied;
Fig. 2 is a block diagram for explaining essential control functions of a server shown in Fig. 1;
Fig. 3 is a view showing an example of a set of medical information that is supplied from the server of Fig. 1 to a member;
Fig. 4 is a flow chart representing a portion of the essential control functions of the server of Fig. 1, i.e., a virtual-hospital controlling routine; and
Fig. 5 is a flow chart representing another portion of the essential control functions of the server of Fig. 1, i.e., a health-related-information-supply controlling routine.

### DETAILED DESCRIPTION OF PREFERRED EMBODIMENTS

Hereinafter, there will be described a preferred embodiment of the present-invention in detail by reference to the drawings.

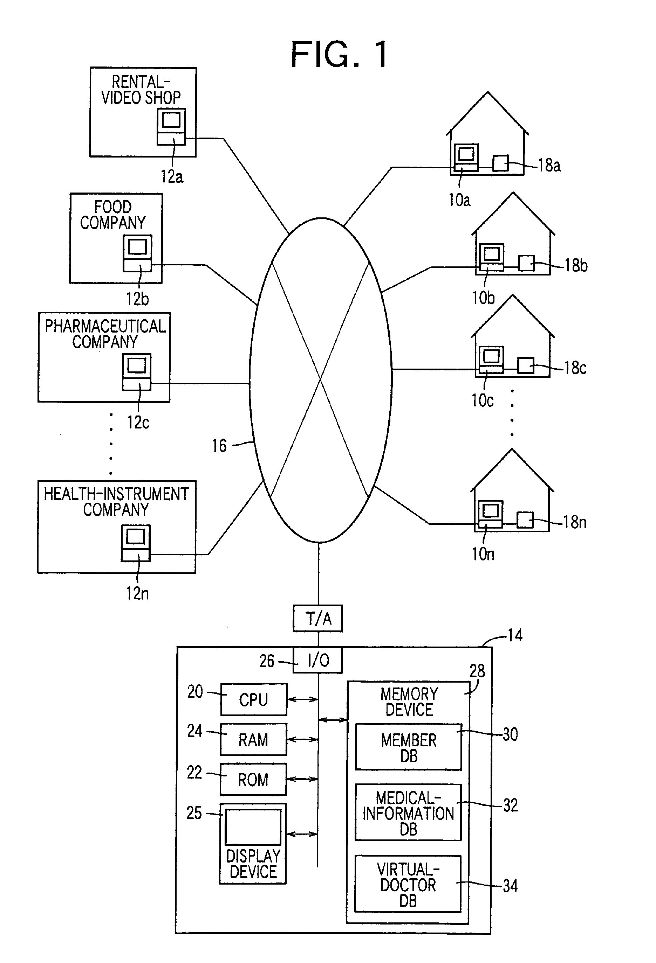

Fig. 1 is a view for explaining the construction of a medical-information and health-related-information supplying system as a health-related-information supplying apparatus to which the present invention is applied. As shown in Fig. 1, the present information supplying system includes a plurality of stationary-type or portable-type member's terminal devices 10a, 10b, 10c, ..., 10n that are operable by respective living persons as respective members. The member's terminal devices may be television sets, personal computers, or portable telephones that have the function of making communications via the internet. The information supplying system additionally includes a plurality of dealer's terminal devices 12a, 12b, 12c, ..., 12n that are operable by respective dealers belonging to, e.g., food companies, pharmacies or pharmaceutical companies, fitness clubs, health-instrument sales companies, rental-video shops; and a server 14 as a virtual hospital that is provided in an information supplying company. The information supplying system further includes a communication line 16, such as wire or wireless internet (i.e., communication network) or wire or wireless telephone line, that connects the member's terminal devices 10n, the dealer's terminal devices 12n, and the server 14, with one another, so that highly secret codes, such as SSL (secure sockets layer), can be communicated among those elements 10n, 12n, 14. The member's terminal devices 10a, 10b, 10c, ..., 10n and the dealer's terminal devices 12a, 12b, 12c, ..., 12n are provided by, e.g., personal- computers each including a display device, and the server 14 is provided by, e.g., a high-speed and high-capacity electronic computer.

A plurality of physical-information obtaining devices 18a, 18b, 18c, ..., 18n are connected to the plurality of member's terminal devices 10a, 10b, 10c, ..., 10n, respectively. Each of the physical-information obtaining devices 18 periodically obtains, from a corresponding one of the members (i.e., living persons), various sorts of physical information including blood pressure BP, weight W, heart rate HR, electrocardiograph waveform ECG, temperature TB, autonomic-nerve activity, pulse-wave propagation velocity PWV, augmentation index AI, eye sight, blood sugar, allergy, and genes. Each of the physical-information obtaining devices 18a, ..., 18n may include a plurality of sensors that detect the above-indicated various sorts of physical information, respectively, or an input device that is manually operable by a living person to input the above-indicated various sorts of physical information. The-physical-information obtaining devices 18a, ..., 18n supply the thus obtained respective sets of physical information to the member's terminal devices 10n, respectively. The blood pressure BP, e.g., systolic and diastolic blood pressure values, may be detected in such a manner that each of the physical-information obtaining devices 18a, ..., 18n employs an inflatable cuff, the cuff is wound around an upper arm of the corresponding living person, and blood pressure values of the person are determined, according to so-called oscillometric method, based on change of respective amplitudes of respective heartbeat-synchronous pulses of a cuff pulse wave detected as a pressure signal by a pressure sensor from the cuff during changing of the pressing pressure of the cuff. Alternatively, the blood pressure may be obtained in such a manner that blood pressure values measured using a common sphygmomanometer from the person are inputted through operation of keys of an input device. The weight W may be detected in such a manner that each of the physical-information obtaining devices 18a, ..., 18n employs a weight sensor, and a weight of the person is detected by the weight sensor, or alternatively the weight W may be obtained in such a manner that a weight measured using a common scales from the person is inputted through operation of keys of an input device. The heart rate HR may be calculated based on the number of heartbeat-synchronous pulses of the above-described cuff pulse wave that are produced in unit time, or based on the number of heartbeat-synchronous pulses of an electrocardiograph waveform that are produced in unit time and are detected by an electrocardiograph device, not shown. The electrocardiograph waveform ECG may be detected by an electrocardiograph device, not shown. The temperature TB may be detected by a temperature sensor, not shown, that is supported by the above-described cuff and is worn with the cuff on the person. The autonomic-nerve activity may be determined based on fluctuations of the heart rate values HR or the blood pressure values BP. The pulse-wave propagation velocity PWV may be determined based on a propagation time from the time when a second heart sound II is detected by a heart-sound microphone to the time when a dicrotic notch of a carotid pulse wave is detected, and a propagation distance between the heart and the carotid artery. The augmentation index AI may be defined as a ratio or proportion of one of an amplitude of an incident-wave component of an arterial pulse wave, such as carotid pulse wave, and an amplitude of a reflected-wave component of the pulse wave, to the other, and may be determined based on the waveform of the carotid pulse wave, or the cuff pulse wave.

The server 14 is essentially provided by a so-called microcomputer including a CPU (central processing unit) 20, a ROM (read only memory) 22 that stores control programs, a RAM (random access memory) 24 that functions as a temporary storage device, a display device 25, and an input-and-output interface 26 that is connected via a terminal adaptor TA to the communication line 16. The server 14 includes a memory device 28 that stores various sorts of data bases (DB) corresponding to various sorts of information. More specifically described, the memory device 28 includes a member DB (data base) 30 that stores respective names of the living persons who have contracted, at their own charge or no charge, with the information supplying company to become, in advance, members who are to be supplied with medical information from the company; respective identification codes ID of the members; respective pass codes of the members; and respective names and identification codes ID of the dealers. The memory device 28 additionally includes a medical-information DB (data base) 32 that stores respective sets of medical information including respective sets of physical information sent from the members, and respective medical evaluations or diagnoses automatically made based on those sets of physical information. The memory device 28 further includes a virtual-doctor DB (data base) 34 that stores a diagnosing program for use in automatically making those medical evaluations or diagnoses on the sets of physical information or respective changes (or trends) of the sets of information. The virtual-doctor DB 34 additionally stores sets of health-related information, such as diet information or hypertension-related information, that are produced based on the sets of physical information or the respective changes or trends of the sets of physical information so as to treat obesity or hypertension.

Thus, the member's terminal devices 10n are operated by the respective members who are registered in the server 14, and each one of the member's terminal devices 10n periodically sends a set of physical information of a corresponding one of the members to the server 14. At that time, the server 14 stores the set of physical information, and automatically makes, according to the diagnosing program stored in the virtual-doctor DB 34, a medical evaluation or diagnosis on the set of physical information or the change or trend of the current set of physical information from the past sets of physical information stored in the medical-information DB 32. In addition, the server 14 sends the thus made medical evaluation or diagnosis, together with the current and past sets of physical information of the member, to the member's terminal device 10n, so that the medical evaluation or diagnosis and the sets of physical information are displayed on the display device of the terminal device 10n of the member. In addition, each of the member's terminal devices 10n may be operated by a corresponding one of the members to request the server 14 to send one or more sets of health-related information, such as diet-related information to decrease weight or hypertension-related information to lower blood pressure. At that time, the server 14 supplies, to the respective terminal devices 12n of the dealers who are registered in advance in the server 14 and have respective different fields, the sort or sorts of health-related information requested, and the set of medical information related to the member and stored in the medical-information DB 32, in such a manner that the name of the member is unidentified. This supplying may be carried out either directly via, e.g., electronic mails, or indirectly via, e.g., an electronic bulletin board. When each of the dealers obtains the sort or sorts of health-related information requested and the set of medical information of the member unidentified, the each dealer selects, from its commercial items such as goods, one or more items that are the most suitable for the sort or sorts of health-related information requested and the set of medical information of the unidentified member, and sends a set of health-related information presenting the selected commercial item or items, to the server 14 via, e.g., an electronic mail. The respective sets of health-related information received by the server 14 from the dealers. are sent to the terminal device 10n of the member who has requested, so that the sets of health-related information are displayed on the terminal device 10n.

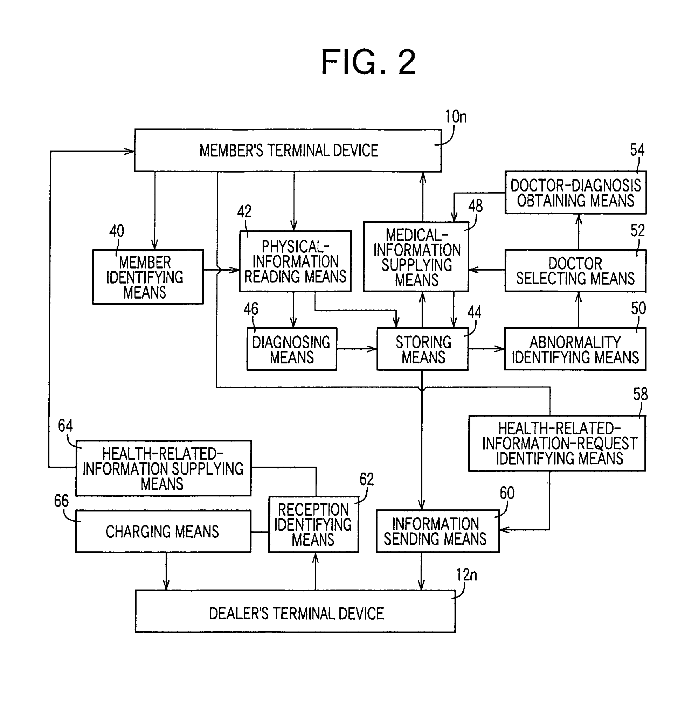

Fig. 2 is a block diagram for explaining the essential control functions of the above-described server 14. A member identifying means 40 judges whether an identification code ID inputted and sent by an arbitrary one of the member's terminal devices 10n is identical with one of the identification codes ID pre-stored in the member DB 30, and thereby judges whether a living person who has made access to the server 14 is one of the members pre-registered in the server 14. A physical-information reading means 42 reads, when the member identifying means 40 has identified the person as one of the registered members, the set of physical information sent by the member's terminal device 10n, and a storing means 44 stores, in the medical-information DB 32, the thus read set of physical information in such a manner that the set of physical information read is associated with the identification code ID of the member identified. A diagnosing means 46 automatically makes a diagnosis on the set of physical information read by the reading means 42, according to the diagnosing program pre-stored in the virtual-doctor DB 34. For example, the diagnosing means 46 judges whether the set of physical information read by the reading means 42 falls in a predetermined reference range, and thereby makes a medical evaluation or diagnosis. The storing means 44 stores, in the medical-information DB 32, the thus made medical evaluation or diagnosis such that the medical evaluation or diagnosis is associated with the identification code ID or the set of physical information. A medical-information supplying means 48 supplies, to the member's terminal device 10n that has sent the current set of physical information, the current and past sets of physical information, and the medical evaluation or diagnosis made on those sets of physical information, all of which have been stored by the storing means 44 in the medical-information DB 32, so that the trend of physical information and the medical evaluation or diagnosis are displayed on the member's terminal device 10n.

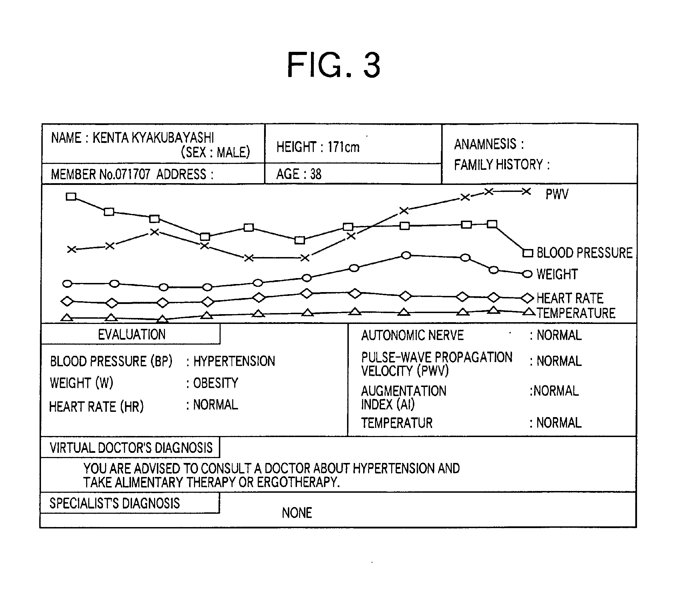

An abnormality identifying means 50 judges whether each set of physical information read by the reading means 42, or an amount of change of the set of physical information, falls outside a predetermined normal range and thereby judges whether each member is abnormal. The normal range may be identical with the above-described reference range. A doctor selecting means 52 selects, when the abnormality identifying means 50 judges based on the set of physical information that the member is abnormal, the most appropriate one of the doctors who are listed and stored in the memory device 28, based on the sort of the physical information judged as abnormal, and an address of the abnormal member, such that the selected doctor is specialized in the sort of the abnormality and is near to the address of the abnormal member. A doctor-diagnosis obtaining means 54 sends the current set of physical information judged as abnormal, and the past sets of physical information and the automatic evaluation or automatic diagnosis stored in the medical-information DB 32, to the doctor selected by the doctor selecting means 52, requests the doctor to make a diagnosis on the current set of physical information, and receives the diagnosis made by the doctor. The storing means 44 stores, in the medical-information DB 32, the diagnosis made by the doctor, such that the diagnosis is associated with the identification code ID or the physical information, and the medical-information supplying means 48 sends the diagnosis to the member. Fig. 3 shows an example of a set of medical information that is displayed on the display device of the member's terminal device 10n that has sent the set of physical information to the server 14. In this way, each member can send his or her own physical information via the member's terminal device 10n, so as to obtain the automatically made diagnosis and additionally obtain, when the physical information is abnormal, the diagnosis made by the actual doctor on the physical information. Thus, the member can go to the "virtual" hospital to take the medical examination, and even if the member may live at a remote place from the virtual hospital or the server 14, the member can obtain the results as if he or she had take an actual examination at an actual hospital.

A health-related-information-request identifying means 58 identifies a health-related-information request sent to the server 14 from an arbitrary one of the user's terminal devices 10n. When the health-related-information-request identifying means 58 has identified a health-related-information request sent from one of the user's terminal devices 10n, an information sending means 60 selects,. from the medical-information DB 32, the set of medical information (including the automatic diagnosis) related to the member, and supplies, to the respective terminal devices 12n of the dealers pre-registered in the server 14, the sort or sorts of health-related information requested and the selected set of medical information, via either the electronic bulletin or the respective electronic mails. This supplying is carried out such that the name of the member is unidentified. To this end, the information identifying the member, such as the name, address, or electronic-mail address of the member, is removed from the information supplied to the dealers, in particular, the set of medical information related to the member and pre-stored in the medical-information DB 32.

A reception judging means 62 judges whether the server 14 has received the sets of health-related information sent from the terminal devices 12n of the dealers. A health-related-information supplying means 64 supplies, to the terminal device 10n of the member who has requested, the sets of health-related information the respective receptions of which from the dealers have been judged by the reception judging means 62. This supplying may be carried out via an electronic mail. Simultaneously, a charging means 66 charges the dealers who have sent the respective sets of health-related information. To this end, the charging means 66 sends a set of charging information to the terminal devices 12n of each of the dealers. The charging information may be a bill, or a notice to draw money from a bank account of the each member who contracted with the information supplying company to allow it.

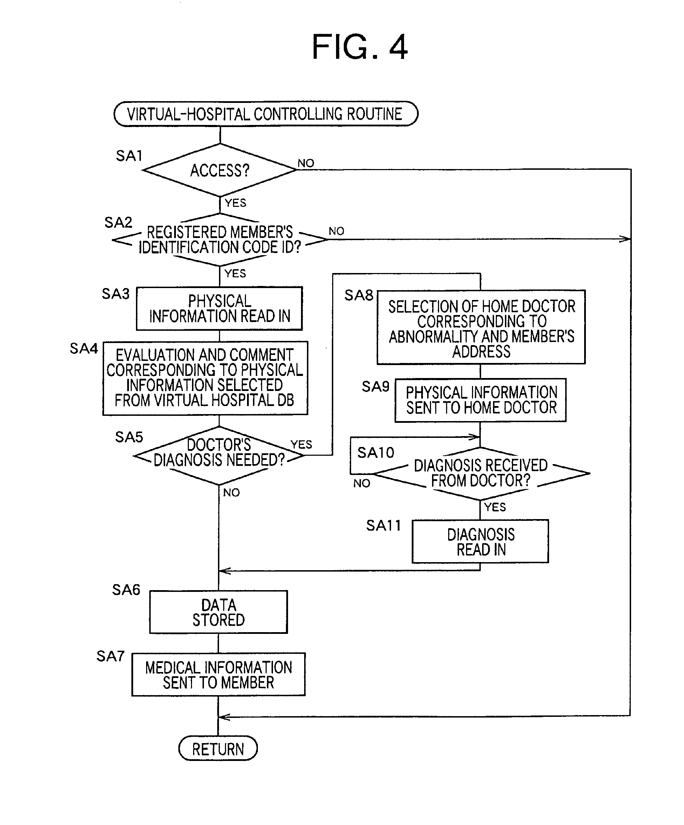

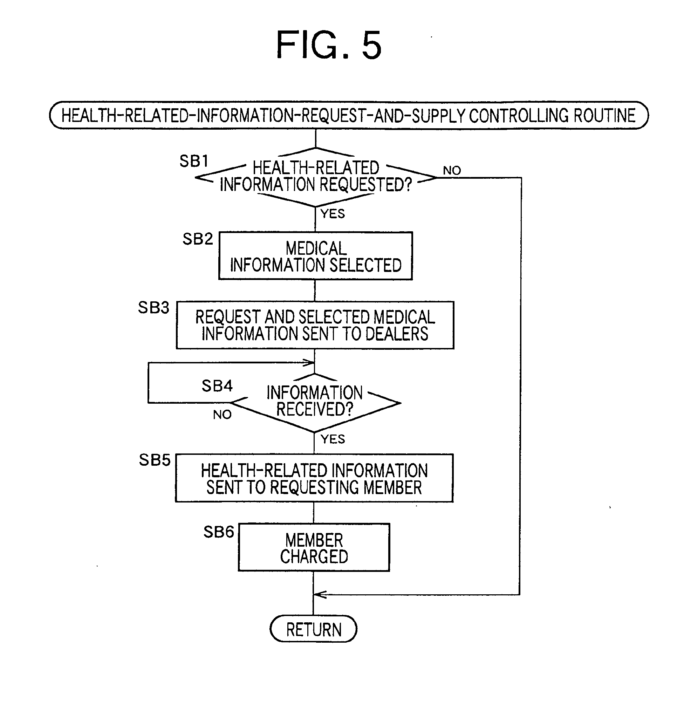

Figs. 4 and 5 show respective flow charts representing the essential control functions of the above-described server 14, i.e., Fig. 4 is a virtual-hospital (i.e., medical-information-supply) controlling routine and Fig. 5 is a health-related-information-supply controlling routine. These routines are iteratively executed at a short period.

At Step SA1 of Fig. 4 (hereinafter, "Step(s)" is omitted), the server 14 judges whether any one of the member's terminal devices 10n has made access to the server 14. If a negative judgment is made at SA1, this routine is quitted. On the other hand, if a positive judgment is made at SA1, the control goes to SA2 corresponding to the member identifying step or the member identifying means 40. At SA2, the server judges whether the member's identification code ID inputted by the member's terminal device 10n is identical with one of the member's identification codes ID pre-registered in the server. If a negative judgment is made at SA2, this routine is quitted. On the other hand, if a positive judgment is made at SA 2, the control goes to SA3 corresponding to the physical-information reading step or the physical-information reading means 42. At SA3, the server reads a set of physical information that has been obtained by the physical-information obtaining device 18n connected to the member's terminal device 10n and has been sent thereto from the terminal device 10n, i.e., blood pressure BP, weight W, heart rate HR, electrocardiograph waveform ECG, temperature TB, autonomic-nerve activity, pulse-wave propagation velocity PWV, augmentation index AI, eye sight, blood sugar, allergy, and genes.

Then, the control goes to SA4 corresponding to the diagnosing step or the diagnosing means 46. At SA4, the server automatically makes, according to the automatically evaluating and diagnosing program pre-stored in the virtual-doctor DB 34, a diagnosis on the new set of physical information, the past set or sets of physical information, and the change of the new and past sets of physical information. For example, the server selects one of a plurality of evaluations and/or comments that corresponds to the new set of physical information. Fig. 3 shows the thus made evaluations and the thus made virtual doctor's diagnosis. Then, the control goes to SA5 corresponding to the abnormality identifying step or the abnormality identifying means 50. At SA5, the server judges whether it is needed to consult an actual doctor or a specialist. More specifically described, the server judges whether the new set of physical information falls outside a predetermined normal range. If a negative judgment is made at SA5, the control goes to SA6 corresponding to the storing step or the storing means 44. At SA6, the server stores, in the medical-information DB 32, the new set of physical information and the automatically made evaluation and/or diagnosis, such that the set of medical information is associated with the identification code ID of the member. Then, the control goes to SA7 corresponding to the medical-information supplying step or the medical-information supplying means 48. At SA7, the server sends, to the member's terminal device 10n that has sent the new set of physical information, the set of medical information that has been stored, for the member, in the medical-information DB 32, that is, the new and past sets of physical information (i.e., the trend of physical information) and the automatic evaluation and/or diagnosis, so that the set of medical information is displayed on the display device of the member's terminal device 10n, as shown in Fig. 3. Thus, each member can easily and economically request the virtual hospital or doctor to check his or her physical condition or make a diagnosis on it.

On the other hand, if a positive judgment is made at SA5, the control goes to SA8 corresponding to the doctor selecting step or the doctor selecting means 52. At SA8, the server selects, from the pre-stored list of doctors, a doctor or a specialist who is appropriate for the sort of the abnormal physical information, the degree of abnormality of the physical information, and the address of the member. For example, a specialist who is specialized in the sort of the abnormal physical information, or a doctor belonging to a medical institution located in an area where the member can go. Next, the control goes to SA 9 to SA11 corresponding to the doctor-diagnosis obtaining step or the doctor-diagnosis obtaining means 54. First, at SA9, the server sends, to the selected doctor, the abnormal, new set of physical information of the member and the past sets of physical information of the same, and requests the doctor to make a diagnosis on the sets of physical information. Then, at SA10, the server judges whether the server has received a diagnosis sent from the doctor. SA10 is repeated until a positive judgment is made. When a positive judgment is made at SA10, the control goes to SA11 to read in the diagnosis sent from the doctor. Subsequently, the control goes to SA6 and SA7. At SA6, the server stores, in the medical-information DB 32, not only the new set of physical information and the automatic evaluation or diagnosis, but also the diagnosis made by the actual doctor, such that the set of medical information is associated with the identification code ID of the member. Then, the control goes to SA7 corresponding to the medical-information supplying step or the medical-information supplying means 48. At SA7, the server sends, to the member's terminal device 10n that has sent the new set of physical information, the set of medical information that has been stored, for the member, in the medical-information DB 32, that is, the new and past sets of physical information (i.e., the trend of physical information), the automatic evaluation or diagnosis, and the diagnosis made by the actual doctor, so that the set of medical information is displayed on the member's terminal device 10n, as shown in Fig. 3, and additionally the diagnosis made by the actual doctor, and the name and address of the hospital to which the doctor belongs, or the address of the doctor are indicated in an area prepared therefor (i.e., the lowermost area). Thus, each member can easily and economically request the virtual hospital or doctor to check his or her physical condition or make a diagnosis on it, and additionally can obtain a diagnosis made by the actual doctor when the new set of physical information is judged as abnormal. If necessary, the member can request the doctor to re-examine his or her physical condition.

Then, the control goes to SB1 of Fig. 5 corresponding to the health-related-information-request identifying step or the health-related-information-request identifying means 58. At SB1, the server judges whether an arbitrary one of the respective user's terminal devices 12n has sent, to the server 14, a request to send one or more sets of health-related information. This request is, for example, "Since I want to decrease my weight, please teach me a diet food. However, recently, my blood pressure has ..., I have egg allergy, my weight has increased by ... in the last three months." If a negative judgment is made at SB1, this routine is quitted. On the other hand, if a positive judgment is made at SB1, the control goes to SB2 and SB3 corresponding to the information sending step or the information sending means 60. First, at SB2, the server selects, from the sets of medical information pre-stored in the medical-information DB 32, the set of medical information (including the automatic diagnosis) related to the member who has sent the request. Subsequently, at SB3, the server supplies, to the respective terminal devices 12n of the pre-registered dealers, the set of medical information selected at SB2 and the sort or sorts of health-related information requested by the member, via an electronic bulletin or respective electronic mails, in such a manner that the member is unidentified.

Then, the control goes to SB4 corresponding to the reception judging step or the reception judging means 62. At SB4, the server judges whether the server has received the sets of health-related information sent from the respective terminal devices 12n of the dealers. Each set of health-related information includes the name of the corresponding dealer and the address to which an order is directed, and thus functions as a sort of sales-promoting advertisement. For example, a rental-video company sends, as a set of health-related information, a message "You are advised to see the movie of..., and listening the music of ... is effective to decrease your weight"; a food company sends a message "The special price of 5,000 yen for Diet Supplement A"; a pharmaceutical company sends a message "You are advised to drink this tea to lower your blood pressure and decrease your weight"; and a health-instrument sales company sends a message "Since your blood pressure is too high, it is risky for you to do exercise and we do not have instruments appropriate for you." Step SB4 repeated until a positive judgment is made. Then, the control goes to SB5 corresponding to the health-related-information supplying step or the health-related-information supplying means 64. At SB5, the server supplies, to the terminal device 10n of the member who has requested, the sets of health-related information the respective receptions of which from the dealers have been judged at SB4. Next, the control goes to SB6 corresponding to the charging step or the charging means 66. At SB6, the server sends charging information to the terminal device 12n of each of the dealers, so as to charge the each dealer who has sent the set of health-related information to the server, because the member will purchase, with high possibilities, the dealers' commercial item while taking into account the set of health-related information sent to the member based on his or her own medical information.

As is apparent from the foregoing description of the illustrated embodiment, when the health-related-information-request identifying step SB1 or the health-related-information-request identifying means 58 identifies a health-related-information request sent from one of the pre-registered members, the information sending step SB3 or the information sending means 60 supplies, to the respective terminal devices 12n of the pre-registered dealers, the sort or sorts of health-related information requested by the member and the set of medical information related to the member and stored in the server, via respective electronic mails or an electronic bulletin, in such a manner that the member is not unidentified. When the reception judging step SB4 or the reception judging means 62 judges that the server has received, from the dealers, the respective sets of health-related information corresponding to the sort or sorts of health-related information requested and the set of medical information, the health-related-information supplying step SB5 or the health-related-information supplying means 64 supplies the sets of health-related information to the member. Thus, the server can easily and quickly supply the sets of health-related information suited for the medical information of each member, to the member, in response to the request from the member. In addition, since the medical information of each member is sent anonymously to the dealers, the secrecy of medical information of each member is not damaged.

In addition, in the illustrated embodiment, the medical-information supplying means 48 (SA7) selects, based on the set of physical information sent by one member through the member's terminal device 10n, the set of medical information pre-stored in the server 14 and related to the set of physical information, and sends the selected set of medical information to the member; and the information storing means 44 (SA6) stores the set of physical information of the member inputted through the member's terminal device 10n and the set of medical information supplied to the member. Thus, the server can easily store the respective sets of physical information, and the respective sets of medical information, of the members, on one hand, and, when each member requests a set or sets of health-related information, the server can send, to the dealers, the set of medical information (including the set of physical information) stored by the information storing means 44, on the other hand. Thus, the server can supply more appropriate sets of health-related information for the member, to the member.

In the illustrated embodiment, the diagnosing means 46 (or the diagnosing step SA5) automatically makes a diagnosis on a physical condition of the member, based on the set of physical information of the member inputted through the member's terminal device 10n; and the information sending means 62 or the information sending step SB3 sends the set of medical information including the diagnosis made by the diagnosing means 46. That is, when each member inputs his or her physical information to obtain his or her medical information from the server 14, the diagnosing means 46 automatically makes a diagnosis based on the physical information of the member, and this diagnosis is sent with the set of medical information to the dealers at the information sending step SB3. Thus, each dealer can supply a more appropriate set or sets of health-related information for the requesting member, to the member.

While the present invention has been described in its embodiment by reference to the drawings, it is to be understood that the present invention can otherwise be embodied.

For example, in the illustrated embodiment, the server 14 is described such that the server 14 is provided by a single computer. However, the server may be provided by a plurality of computers, and those computers may be provided at respective positions remote from each other.

In the illustrated embodiment, each of the physical-information obtaining devices 18n connected to the member's terminal devices 10n has the functions of obtaining blood pressure BP, weight W, heart rate HR, electrocardiograph waveform ECG, temperature TB, autonomic-nerve activity, pulse-wave propagation velocity PWV-, augmentation index AI, etc. However, each physical-information obtaining device 18n may be one that can obtain only a portion (one, two, ..., but not all) of the above-indicated sorts of physical information, or one that can obtain other sorts of physical information. The sorts of physical information that are not directly detected by each physical-information obtaining device 18n may be manually inputted through each member's terminal device 10n after having been measured using scales, a sphygmomanometer, a heart-rate meter, an electrocardiograph, etc. In this sense, each physical-information obtaining device 18n may be one that does not include any sensors.

In the flow charts shown in Figs. 4 and 5, the order of the steps may be changed, as needed. In addition, SA5 to SA11 of Fig. 4 may be omitted.

While the present invention has been described in detail in its embodiments by reference to the drawings, it is to be understood that the present invention is not limited to the details of the described embodiments but may be embodied with various changes or improvements that may occur to a person skilled in the art.

## Claims

1. A method of supplying, from a server which is connected via a communication line to a plurality of member's terminal devices which are respectively operated by a plurality of members who are registered in advance in the server, at least one set of health-related information based on a set of medical information related to one of the members and stored in the server, to said one member, in response to a health-related-information request sent to the server from said one member, the method comprising:
a health-related-information-request identifying step of identifying said health-related-information request sent to the server from said one member,
an information sending step of sending, when said health-related-information request sent from said one member has been identified, said health-related-information request sent from said one member, and the set of medical information related to said one member and stored in the server, to at least one dealer who is registered in advance in the server, in a manner that said one member is unidentified, so as to request the dealer to send the set of health-related information, and
a health-related-information supplying step of receiving the set of health-related information sent from the dealer in response to the health-related-information request and the set of medical information sent to the dealer, and supplying the received set of health-related information to said one member.

2. A method according to claim 1, further comprising
a medical-information supplying step of selecting, from a plurality of sets of medical information respectively related to the plurality of members and stored in the server, the set of medical information related to a set of physical information of said one member inputted through a corresponding one of the member's terminal devices, and sending the selected set of medical information, to said one member, and
an information storing step of storing the set of physical information of said one member inputted through said one member's terminal device and adding the set of physical information to the selected set of medical information.

3. A method according to claim 1 or claim 2, further comprising a diagnosing step of making a diagnosis about a physical condition of said one member based on the set of physical information of said one member sent to the server from said one member's terminal device, wherein the information sending step comprises sending, to the dealer, the selected set of medical information including the made diagnosis.

4. A medical-information supplying apparatus including a server (14) which is connected via a communication line (16) to a plurality of member's terminal devices (10) which are respectively operated by a plurality of members who are registered in advance in the server, and supplying, from the server, a set of health-related information based on a set of medical information related to one of the members and stored in the server, to said one member, in response to a health-related-information request sent to the server from said one member, the apparatus comprising:
a health-related-information-request identifying means (58) for identifying said health-related-information request sent to the server (14) from said one member;
an information sending means (60) for sending, when said health-related-information-request identifying means has identified said health-related-information request sent from said one member, said health-related-information request sent from said one member, and the set of medical information related to said one member and stored in the server (14), to at least one dealer who is registered in advance in the server, in a manner that said one member is unidentified, so as to request the dealer to send the set of health-related information, and
a health-related-information supplying means (62, 64) for receiving the set of health-related information sent from the dealer in response to the health-related-information request and the set of medical information sent to the dealer by the information sending means (60), and supplying the received set of health-related information to said one member.

5. An apparatus according to claim 4, further comprising:
a medical-information supplying means (48) for selecting, from a plurality of sets of medical information respectively related to the plurality of members and stored in the server (14), the set of medical information related to a set of physical information of said one member inputted through a corresponding one of the member's terminal devices (10), and sending the selected set of medical information, to said one member; and
an information storing means (44) for storing the set of physical information of said one member inputted through said one member's terminal device and adding the set of physical information to the selected set of medical information.

6. An apparatus according to claim 4 or claim 5, further comprising a diagnosing means (46) of making a diagnosis about a physical condition of said one member based on the set of physical information of said one member sent to the server (14) from said one member's terminal device, wherein the information sending means (60) sends, to the dealer; the selected set of medical information including the made diagnosis.

7. An apparatus according to any of claims 4 to 6, further comprising a plurality of physical-information obtaining devices (18) which are connected to the member's terminal devices (10), respectively, and which obtain respective sets of physical information from the members and supply the respective sets of physical information to the member's terminal devices.

8. An apparatus according to any of claims 4 to 7, wherein each of the member's terminal devices (10) comprises a display device which displays the set of health-related information supplied from the health-related-information supplying means (64).
